(19) **Europäisches Patentamt / European Patent Office / Office européen des brevets**

(11) **EP 3 636 194 B1**

(12) **EUROPEAN PATENT SPECIFICATION**

(45) Date of publication and mention
of the grant of the patent:
**06.03.2024 Bulletin 2024/10**

(21) Application number: **18805766.5**

(22) Date of filing: **21.05.2018**

(51) International Patent Classification (IPC):
**A61B 34/20** (2016.01)    **A61B 34/37** (2016.01)
**A61B 1/00** (2006.01)    **A61B 34/00** (2016.01)
**A61B 34/35** (2016.01)    **A61B 34/30** (2016.01)
**A61B 34/10** (2016.01)    **A61B 90/00** (2016.01)

(52) Cooperative Patent Classification (CPC):
**A61B 34/20; A61B 34/30; A61B 90/37;**
A61B 1/00149; A61B 34/25; A61B 2034/2059;
A61B 2034/301; A61B 2090/061; A61B 2090/365;
A61B 2090/373

(86) International application number:
**PCT/CN2018/087645**

(87) International publication number:
**WO 2018/214840 (29.11.2018 Gazette 2018/48)**

(54) **SURGICAL ROBOT SYSTEM, AND METHOD FOR DISPLAYING POSITION OF SURGICAL INSTRUMENT**

CHIRURGISCHES ROBOTERSYSTEM UND VERFAHREN ZUR ANZEIGE DER POSITION EINES CHIRURGISCHEN INSTRUMENTS

SYSTÈME DE ROBOT CHIRURGICAL ET PROCÉDÉ D'AFFICHAGE DE LA POSITION D'UN INSTRUMENT CHIRURGICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2017 CN 201710385151**

(43) Date of publication of application:
**15.04.2020 Bulletin 2020/16**

(73) Proprietor: **Shanghai Microport Medbot (Group) Co., Ltd.**
**Shanghai 201203 (CN)**

(72) Inventors:
• **GAO, Xu**
  **Shanghai 201203 (CN)**
• **SHI, Yunlei**
  **Shanghai 201203 (CN)**
• **HE, Chao**
  **Shanghai 201203 (CN)**
• **WANG, Jiayin**
  **Shanghai 201203 (CN)**
• **ZHU, Xiang**
  **Shanghai 201203 (CN)**
• **JIANG, Yizhi**
  **Shanghai 201203 (CN)**

(74) Representative: **Patentship Patentanwaltsgesellschaft mbH**
**Elsenheimerstraße 65**
**80687 München (DE)**

(56) References cited:
EP-A2- 2 046 538    CN-A- 102 860 841
CN-A- 105 025 787    CN-A- 105 078 576
CN-A- 105 899 143    CN-A- 107 049 492
US-A1- 2011 306 986    US-A1- 2018 228 343

## Description

## TECHNICAL FIELD

The present disclosure relates to the technical field of medical instruments, and in particular, to a surgical robot system, and a method for displaying position of a surgical instrument.

## BACKGROUND

Since the first application of robotics to cerebral puncture surgery under CT navigation in the 1980s, surgical robotics has rapidly developed and expanded. Nowadays, robotic surgery is widely used. According to statistics, the number of robotic surgery cases per year in the world exceeds 520,000. Robotic minimally invasive surgery is the most important development direction of robotic surgery. In the field of urinary surgeries in the United States. More than 50% of patients actively choose robotic minimally invasive surgery, and more than 90% of radical prostatectomy is achieved by using minimally invasive surgical robots. Benefit from the latest developments in clinical medicines, robotics, mechanisms, computer control technology, materials science, digital diagnostics, etc., the robotic minimally invasive surgery technology not only supports the more complex minimal invasion surgeries, the more accuracy and quality of surgical operations and the safer surgery, but also bring various digital surgical diagnosis information, computer-aided judgment and monitoring, as well as real-time operational assessment and constraints in surgical operations, which make surgical operations to a new stage of development, based on the combination of the powerful information processing and judgment ability of the computer and the rich experience of surgeons.

Compared with the conventional open surgery and the laparoscopic minimally invasive surgery, robotic minimally invasive surgery has the following advantages.

(1) More flexible tool terminal: a wrist with multi-degree of freedom is added to the terminal of the surgical tool to achieve the multi-degree of freedom kinematic ability in a narrow space, which fundamentally improves the kinematic flexibility and operability of the end of the surgical tool, thereby improving the adaptability and safety of the surgical robot for complex surgery.

(2) More precious surgical operation: the use of computer control technology, servo control technology, and precision electromechanical transmission technology enables the minimally invasive surgery robot to drive the terminal of the surgical tool to move with higher precision and stability. So the robotic operation allows more accurate motion and higher quality than manual operation.

(3) High-definition stereoscopic vision: the stereoscopic endoscope and the 3D stereoscopic imaging system are used to establish high-definition stereoscopic visual feedback of the surgical scene. It enables the surgeon to obtain depth information of the movement and operation, and also feel the environmental information of the surgical scene in an immersive manner, so that the surgeon can clearly and accurately perform tissue localization and instrument operation.

(4) Intuitive operation: the use of the master-slave operation mode enables the surgeon to operate intuitively like an open surgery. The robot system automatically detects the movement of the surgeon's hand and controls the surgical tool to move like the surgeon's actions. Such the operation mode avoids the trouble that the anti-lever operation brings to the surgeon, reduces the difficulty of the surgical operation, improves the comfort of the surgical operation, and further increases the surgical effect and quality stability.

At present, some defects exist in the use of surgical robots that are widely used in clinical practice. For example, the field of view of the endoscope is small, which results in a relatively smaller viewing range of the surgeon during operation, causing a decrease in operation comfort and operation speed. More seriously, when a plurality of surgical instruments are simultaneously used for surgery at the same time, the surgeon cannot see all the surgical instruments due to the narrow field of view of the endoscope. As a result, the surgeon cannot clearly find out the position of each surgical instrument during the surgery and lack a global sense, affecting the operation speed and the operation efficiency. And even in severe case, vulnerable organs may be touched unexpectedly by the surgical instrument, affecting the surgery safety, and even causing surgery failure. In particular, surgeons often need to suspend surgery to switch to endoscopic control to move or enlarge the field of view of the endoscope to find surgical instruments that are not in the field of view, seriously affecting the operation speed.

In order to solve the problem that a collision may occur between the surgical instruments during the operation of the surgeon since all the surgical instruments cannot be displayed due to the narrow field of view of the endoscope, the Chinese Patent Publication No. CN102448680A discloses a robotic surgical system, including: a robot including a linkage supporting at least one surgical tool; a kinematic component coupled to the robot to obtain kinematic position information associated with the linkage; a display; and a first component configured to couple the display to the kinematic component to display a synthetic representation of the robot, the synthetic representation including a graphical three-dimensional model of at least a portion of the link. According to an embodiment of the patent, an overall three-dimensional composite image of the patient side cart (including various robot components) is shown by a viewer or display, and the endoscopic view can be superimposed on the composite image. The composite image enables all components of the patient side cart to be viewed via the viewer, thus allowing the surgeon to monitor the movement of the robot and the tool. In addition, these components are observed by combining the composite image with the endoscope view, so that the surgeon has a good understanding of the field of view of the endoscope in space, thereby avoiding collisions between components. However, the composite image displayed in real time by the display of the patent is large and complex in content, and contains three-dimensional images of all robot components and tools, which is not conducive to surgeon's observation. Moreover, in order to generate the composite image, the computing capability requirement for the processor is high, which increases the costs of robotic surgical system.

In document EP 2 046 538 A2 a tool position and identification indicator displayed in a boundary area of a computer display screen is disclosed.

Therefore, there is a need for a system and method that can display positions of surgical instruments outside the field of view of the endoscope in a more compact and clear manner.

## SUMMARY OF THE DISCLOSURE

The invention is defined in the appended set of claims.

Some embodiments provide a surgical robot system and a method for displaying position of a surgical instrument, and in particular, in the case where a plurality of surgical instruments are present, the problem of how to display position information of the surgical instrument in an endoscope perspective in a simple and visual manner can be solved.

And the surgical robot system comprises an image arm, a tool arm, a display unit, and a computing unit, wherein

the image arm is configured to connect to an endoscope, the endoscope being configured to collect image information;
the tool arm is a remote-center-of-motion mechanism and configured to connect to a surgical instrument;
the surgical instrument comprises at least one feature point identifying position information of the surgical instrument;
the display unit is communicatively connected to the computing unit;
wherein a terminal of the endoscope is defined with an endoscope coordinate system;
the computing unit is configured to obtain a coordinate value of the feature point in the endoscope coordinate system according to a kinematics equation, obtain position information of the surgical instrument in an endoscope lens plane according to the coordinate value of the feature point, and perform graphical processing on the position information; and
the display unit receives and displays the graphically processed position information.

The feature point comprises a first feature point and a second feature point; the first feature point is a surgical instrument endpoint, and the second feature point is a point on the surgical instrument corresponding to a position of a remote-center-of-motion point

Optionally, the step of performing graphical processing on the position information by the computing unit comprises: using a first identifier to represent the position information of the surgical instrument in the endoscope lens plane.

Optionally, the surgical instrument has identification information; the computing unit obtains image information collected by the endoscope, and identifies whether the surgical instrument is in the field of view of the endoscope according to the identification information of the surgical instrument.

Optionally, the surgical robot system comprises at least two surgical instruments, wherein different surgical instruments have different identification information; and the first identifier of one or more surgical instruments which are not in the field of view of the endoscope is different from the first identifier of one or more surgical instruments which are in the field of view of the endoscope.

Optionally, the feature point comprises a first feature point, and the first feature point is a surgical instrument endpoint.

Optionally, the position information of the surgical instrument in the endoscope coordinate system is described by a vector line, wherein a starting point of the vector line is an origin of the endoscope coordinate system, and an end point of the vector line is a projection of the first feature point in the endoscope coordinate system in the endoscope lens plane; and the first identifier represents a direction of the vector line.

Optionally, the feature point comprises a first feature point and a second feature point; the first feature point is a surgical instrument endpoint, and the second feature point is a point on the surgical instrument corresponding to a position of a remote-center-of-motion point.

Optionally, the position information of the surgical instrument in the endoscope coordinate system is described by a vector line, wherein a starting point of the vector line is a projection of the second feature point in the endoscope coordinate system in the endoscope lens plane, and an end point of the vector line is a projection of the first feature point in the endoscope coordinate system in the endoscope lens plane; and the first identifier represents a direction of the vector line.

Optionally, the surgical robot system comprises at least two surgical instruments, wherein a length ratio between the first identifiers of the at least two surgical instruments is equal to a length ratio between the vector lines.

Optionally, the step of performing graphical processing on the position information by the computing unit further comprises:

allocating a first display frame, and placing the first identifier into the first display frame; and
sending the first display frame to the display unit.

Optionally, the step of performing graphical processing on the position information by the computing unit comprises:

using a second identifier to represent the position information; allocating, by the computing unit, a third display frame; and placing information obtained by superimposing the second identifier and the image information collected by the endoscope into the third display frame; and
sending the third display frame to the display unit.

Optionally, the position information of the surgical instrument in the endoscope coordinate system is described by a vector line, and the second identifier represents a direction of the vector line, wherein a starting point of the vector line is an origin of the endoscope coordinate system, and an end point of the vector line is a projection of the first feature point in the endoscope coordinate system in the endoscope lens plane, or

the starting point of the vector line is a projection of the second feature point in the endoscope coordinate system in the endoscope lens plane, and the end point of the vector line is a projection of the first feature point in the endoscope coordinate system in the endoscope lens plane,
wherein the first feature point is a surgical instrument endpoint, and the second feature point is a point on the surgical instrument corresponding to a position of a remote-center-of-motion point.

Optionally, the step of performing graphical processing on the position information by the computing unit further comprises: representing the length of the vector line by numbers and characters.

Optionally, the surgical robot system further comprises a control switch, wherein the control switch is communicatively connected to the computing unit and configured to control whether to display the graphically processed position information on the display unit.

The other embodiments provide a method for displaying position of a surgical instrument, comprising:

solving, by a computing unit according to displacement values of joints of a tool arm, an image arm and a surgical instrument, a kinematics equation to obtain a coordinate value of a feature point in an endoscope coordinate system;

obtaining, by the computing unit, position information of the surgical instrument in an endoscope lens plane according to the coordinate value of the feature point;
performing, by the computing unit, graphical processing on the position information; and
receiving and displaying, by a display unit, the graphically processed position information.

The feature point comprises a first feature point and a second feature point; the first feature point is a surgical instrument endpoint, and the second feature point is a point on the surgical instrument corresponding to a position of a remote-center-of-motion point.

In summary, the present disclosure provides a surgical robot system and a method for displaying position of a surgical instrument, and in particular, in the case where a plurality of surgical instruments are present, to solve the problem of how to display position information of the surgical instrument in an endoscope perspective in a simple and visual manner. Further, the surgical robot system provided by the present disclosure can also solve the problem of how to display the position information of the surgical instrument lost in the field of view of the endoscope in a simple and visual manner when the surgeon cannot directly obtain one or more surgical instruments under the field of view of the endoscope. In this way, the surgeon can clearly find out the positions of the surgical instruments connect to the terminal of all the tool arms during the operation, thereby improving the operation speed and the operation efficiency, especially reducing the probability of touching the vulnerable organs and improving the safety and success rate of the surgical operation.

**BRIEF DESCRIPTION OF THE DRAWINGS**

FIG. 1 is a structural block diagram of a surgical robot system according to some embodiments;
FIG. 2 is a schematic diagram of establishing a spatial rectangular coordinate system at an endoscope endpoint, a surgical instrument endpoint, and a remote-center-of-motion point according to some embodiments;
FIG. 3 is a schematic diagram of a display according to some embodiments, wherein all surgical instruments are in the field of view of the endoscope;
FIG. 4 is a schematic diagram of a display according to some embodiments, wherein some of the surgical instruments are not in the field of view of the endoscope;
FIG. 5 is a schematic diagram of a display according to further embodiments, wherein some of the surgical instruments are not in the field of view of the endoscope; and
FIG. 6 is a flowchart showing the operation of a surgical robot system according to some embodiments.

Description of reference numerals:

1-image arm; 2-tool arm; 3-display unit; 31, 32, 33, 34-graphical display window; 4-computing unit; 5-endoscope; 6, 6', 6"-surgical instrument; 7-second identifier; 8-driver; a-endoscope endpoint; b-surgical instrument endpoint; c- a point disposed on the surgical instrument corresponding to the position of the remote-center-of-motion point; cys1-first coordinate system; cys2-second coordinate system; cys3-third coordinate system.

## DETAILED DESCRIPTION

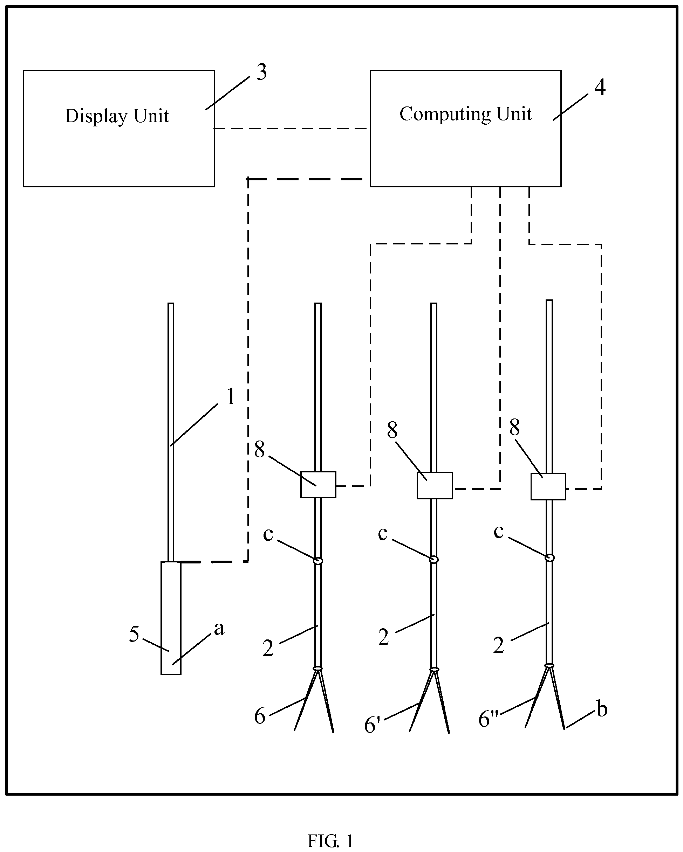

To make the objectives, advantages and features of the present disclosure clearer, the surgical robot system and a method for displaying position of a surgical instrument proposed by the present disclosure will be further described in detail below with reference to FIGs. 1-6. It should be noted that the drawings are in a very simplified form and use non-precise proportions, and are only intended to conveniently and explicitly assist in describing the objectives of embodiments.

As used in the specification and claims, the "tail end" or "distal end" or "terminal" refers to the end distant from an operator of a medical instrument, and the "proximal end" refers to the end near the operator of the medical instrument.

First, referring to FIG. 1, illustrated is a structural block diagram of a surgical robot system according to some embodiments. The surgical robot system comprises an image arm 1, a tool arm 2, a display unit 3, and a computing unit 4. The computing unit 4 is communicatively connected to the display unit 3. In the figure, three surgical instruments are shown. However, the embodiments do not particularly limit the number of surgical instruments, which is mainly set according to actual operation needs. However, for ease of description, the structure of the surgical robot system and the method for displaying position of a surgical instrument are described below in detail by taking three surgical instruments as an example. In addition, the communicative connection relationships between devices are indicated by a dashed line in the drawing.

During the actual operation, the terminal of the image arm 1 can connect to, in particular mount, an endoscope 5 through which collect image information in the surgical environment. The image information includes, but is not limited to, human patient tissue or organ information and position information of the surgical instrument. Moreover, the endoscope 5 is connected to the image arm 1 and communicatively connected to the computing unit 4 in order to display image information in the surgical environment collected by the endoscope 5 in real time. The endoscope 5 is stereoscopic or non-stereoscopic, which is not specifically limited.

In addition, the tool arm 2 is a remote-center-of-motion point mechanism, the terminal of which can connect to, in particular mount, a surgical instrument and drive the surgical instrument to move around a remote-center-of-motion point of the tool arm 2. During operation preparation, a body surface of a patient is placed at the remote-center-of-motion point of the tool arm 2, and a wound is disposed on the body surface of the patient near the remote-center-of-motion point, and then the surgical instrument enters into the human body through the wound to operate the patient tissues or organ. In the some embodiments, the tool arm 2 can be an active remote-center-of-motion point mechanism or a passive remote-center-of-motion point mechanism. Additionally, the surgical instrument includes at least one feature point for identifying position information of the surgical instrument at endoscopic coordinates. The surgical instrument is not particularly limited in the some embodiments, and can be any of needle forceps, surgical scissors, and the like.

In some embodiments, the feature point includes a first feature point and a second feature point. The first feature point may be a surgical instrument endpoint b, and the second feature point may be a point c on the surgical instrument corresponding to the position of the remote-center-of-motion point. It should be understood by those skilled in the art that during the surgical operation, the second feature point on the surgical instrument is not a fixed point. That is, if the surgical instrument is inserted into the human body, the point c on the surgical instrument corresponding to the position of the remote-center-of-motion point is closer to the proximal end of the surgical instrument. On the contrary, if the surgical instrument is retracted close to the body surface, the point c on the surgical instrument corresponding to the position of the remote-center-of-motion point is closer to the distal end of the surgical instrument. For ease of processing, the position information of the remote-center-of-motion point of the tool arm 2 is generally used to represent the position information of the second feature point.

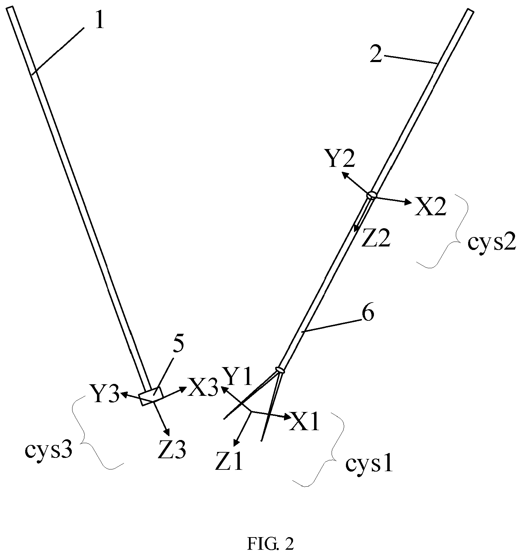

Then, referring to FIG. 2, illustrated is a schematic diagram of establishing a spatial rectangular coordinate system at an endoscope endpoint, a first feature point (i.e., a surgical instrument endpoint), and a second feature point (i.e., the point c on the surgical instrument corresponding to the remote-center-of-motion point) according to some embodiments. In FIG. 2, a first coordinate system cys1 is provided at the first coordinate point, a second coordinate system cys2 is provided at the second feature point, and a third coordinate system cys3 (i.e., an endoscope coordinate system) is provided at the endoscope endpoint a. Since the technique of coordinate system establishment is a technique known to those skilled in the art, those skilled in the art should know how to establish a coordinate system at the point c, the surgical instrument endpoint b, and the endoscope endpoint a by using the computing unit 4 based on the content disclosed in the present application, and the relative position and posture between the coordinate systems are described through transformation matrix.

In some embodiments, the first coordinate system cys1, the second coordinate system cys2, and the third coordinate system cys3 are spatial rectangular coordinate systems and conform to the right-hand rule. The computing unit 4 can be any one of a single chip microcomputer, a DSP, a PLC, a CPLD, and an FPGA, and coordinate systems at the point c, the surgical instrument endpoint b, and the endoscope endpoint a are preset and stored for subsequent coordinate transformation.

Here, in the following description, the relative position and posture between the foregoing three coordinates are further explained by taking the spatial rectangular coordinate system as an example. However, the present disclosure includes, but is not limited to, the spatial rectangular coordinate system.

The origin of the first coordinate system cys1 is set at the surgical instrument endpoint b, and the direction of the Z1 axis of the first coordinate system cys1 may be the axial direction of the surgical instrument 6. Optionally, the positive direction of the Z1 axis may be the direction in which the remote-center-of-motion point c points toward the surgical instrument endpoint b. The direction of the Y1 axis of the first coordinate system cys1 is perpendicular to the axial direction of the surgical instrument 6. Similarly, the specific direction of the Y1 axis is set as needed. And the X1 axis of the first coordinate system cys1 is determined according to the right-hand rule.

The origin of the second coordinate system cys2 is set at the point c, and the direction of the Z2 axis of the second coordinate system cys2 may be the axial direction of the surgical instrument 6. Optionally, the positive direction of the Z2 axis may be the direction in which the point c points toward the terminal of the surgical instrument. In general, the surgical instrument includes an instrument box, a straight bar, and an end effector. The instrument box is connected to a tool arm 2. The straight bar connects the instrument box and the end effector. During operation, the straight bar enters into the human body through the wound (i.e., the remote-center-of-motion point), and the end effector performs the surgical operation in the body. Thus, the direction of the Z2 axis may be the axial direction of the straight bar. The direction of the Y2 axis of the second coordinate system cys2 is perpendicular to the axial direction of the straight bar, but the specific direction of the Y2 axis is set as needed. The X2 axis of the second coordinate system cys2 is determined according to the right-hand rule.

The origin of the third coordinate system cys3 is set at the endoscope endpoint a, and the direction of the Z3 axis of the third coordinate system cys3 may be the perpendicular direction of a mirror surface of the endoscope 5 on the image arm 1. Optionally, the positive direction of the Z3 axis is the direction in which the proximal end of the endoscope 5 (i.e., the portion connected to the image arm 1) points toward the endoscope endpoint a. The Y3 axis of the third coordinate system cys3 is perpendicular to the Z3 axis. Similarly, the specific direction of the Y3 axis is set as needed. And the X3 axis of the third coordinate system cys3 is determined according to the right-hand rule. In this way, the origin of the third coordinate system cys3 is located at the center of the endoscope field of view. Further preferably, for a stereoscopic endoscope, the Y3 axis may extend along a line connecting centers of two mirror surfaces. Similar to the surgical instrument, the endoscope includes a handle and an insertion portion. The insertion portion includes another straight bar connected to the handle, and an optical system, a photoelectric conversion system and the like located at the distal end of the straight bar. The insertion portion enters the human body through another wound of the human body (i.e., the remote-center-of-motion point of the image arm 1) for observing the operation environment.

After the directions of the foregoing three coordinate systems are established, the computing unit 4 can obtain the position descriptions of the first coordinate system cys 1 and the third coordinate system cys3 in the base coordinate by using the kinematics equation, thereby obtaining the position description of the first coordinate system cys1 in the third coordinate system cys3, that is, the position coordinate description of the first feature point (i.e., the surgical instrument endpoint b) relative to the endoscope endpoint a. The kinematics equation described herein does not specifically refer to a certain kinematics equation, and various expressions known in the field of robot kinematics can be applied to the embodiments, as long as the expressions can directly or indirectly calculate the mapping relationship between different coordinate systems according to each joint variable of the surgical robot. Since the embodiments do not aim at the design of the kinematics equation, no further description is made to the specific equations.

Herein, the position coordinate description of the first coordinate system cys1 in the third coordinate system cys3 is defined as the first coordinate value (i.e., the coordinate value of the first feature point), and is represented by the following calculation formula:

$$P_{13} = \begin{bmatrix} x1 \\ y1 \\ z1 \end{bmatrix}$$

Certainly, the computing unit 4 can also obtain the position description of the second coordinate system cys2 in the third coordinate system cys3, that is, the position coordinate description of the second feature point (i.e., the point c on the surgical instrument corresponding to the remote-center-of-motion point) relative to the endoscope endpoint a, by using the kinematics equation. Similarly, the position coordinate description of the second coordinate system cys2 in the third coordinate system cys3 is defined as the second coordinate value (i.e.,

the coordinate value of the second feature point), and is represented by the following calculation formula:

$$P_{23} = \begin{bmatrix} x2 \\ y2 \\ z2 \end{bmatrix}$$

It should be understood that a matrix $P_{13}$ is used to represent the position description of the first feature point in the endoscope coordinate system, and a matrix $P_{23}$ is used to represent the position description of the second feature point in the endoscope coordinate system. $x1$ and $x2$ are coordinate values of the first feature point and the second feature point on the X3 axis of the third coordinate system cys3, respectively. $y1$ and $y2$ are coordinate values of the first feature point and the second feature point on the Y3 axis of the third coordinate system cys3, respectively. $z1$ and $z2$ are coordinate values of the first feature point and the second feature point on the Z3 axis of the third coordinate system cys3, respectively.

Then, after the first coordinate value $P_{13}$ and the second coordinate value $P_{23}$ of the foregoing feature points in the endoscope coordinate system are obtained, the computing unit 4 further obtains the position information of the surgical instrument, in particular the surgical instrument in the body of the patient, in an endoscope lens plane according to the first coordinate value $P_{13}$ and the second coordinate value $P_{23}$. Further, the computing unit 4 performs graphical processing on the position information to obtain graphical information of the position information, and then transmits the graphical information to the display unit 3. Thereafter, the display unit 3 reads the graphical information of the surgical instrument 6 and then displays the graphical information.

In some embodiments, after the first coordinate value $P_{13}$ and the second coordinate value $P_{23}$ are obtained, the computing unit 4 further projects the first coordinate value $P_{13}$ and the second coordinate value $P_{23}$ to the endoscope lens plane, to obtain a coordinate value $P'_{13}$ of the first feature point in the lens plane and a coordinate value $P'_{23}$ of the second feature point in the lens plane, respectively. In some embodiments, the endoscope lens plane is coplanar with an X3Y3 plane (which is a plane formed by the X3 axis and the Y3 axis) under the third coordinate system cys3. Therefore, the coordinate value $P'_{13}$ is expressed as:

$$P'_{13} = \begin{bmatrix} x1 \\ y1 \\ 0 \end{bmatrix}$$

The coordinate value $P'_{23}$ is expressed as:

$$P'_{23} = \begin{bmatrix} x2 \\ y2 \\ 0 \end{bmatrix}$$

In some embodiments, the first feature point and the second feature point may be the endpoint and the starting point of a portion of the surgical instrument inside the human body, respectively. Therefore, a connecting line $\overrightarrow{P'_{23} P'_{13}}$ of coordinate values of the first feature point and the second feature point in the endoscope lens plane can clearly describe the position information of the portion of surgical instrument inside the human body in the endoscope lens plane. Here, the connecting line $\overrightarrow{P'_{23} P'_{13}}$ is a vector line. If there are multiple surgical instruments, the same method can be used to calculate $P'_{13}$ and $P'_{23}$ of two feature points of each surgical instrument. Further, the vector connecting line $\overrightarrow{P'_{23} P'_{13}}$ of the point determined by $P'_{13}$ and the point determined by $P'_{23}$ is displayed in a graphical mode, so that the position relationship between the surgical instruments connected to the terminal of each tool arm in the endoscope lens plane (i.e., in the field of view of the endoscope) can be clearly and intuitively understood.

In some embodiments, various modes for graphically processing the position information of the surgical instrument in the endoscope lens plane are included.

For example, the computing unit 4 allocates at least one display frame in which the graphical information of the position information is loaded. The display unit 3 receives the display frame and displays related content according to the information of the display frame itself (for example, the coordinates and size of the display frame in the display area of the display unit) and the graphical information loaded in the display frame. The computing unit 4 uses the first identifier to indicate the direction of the connecting line. Preferably, the first identifier also indicates the length of the connecting line.

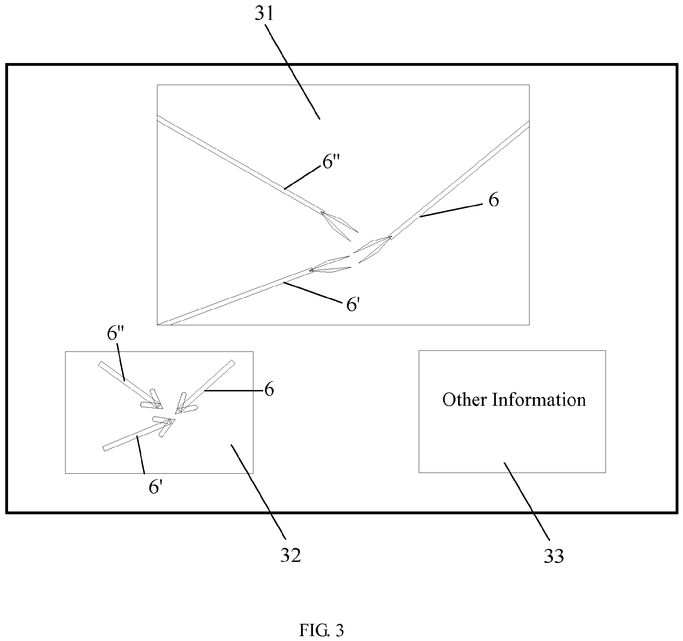

Specifically, as shown in FIG. 3, the surgical robot system includes three tool arms 2. Each tool arm 2 connects to, in particular mounts, one of the surgical instruments 6, 6', 6" (i.e., one tool arm 2 is connected to one surgical instrument), one image arm 1 and a corresponding endoscope 5. The surgical robot system obtains the position information of two feature points of each surgical instrument in the endoscope lens plane by using the computing unit 4. Further, the computing unit 4 uses three line segments with arrows as the first identifiers to indicate the connecting line $\overrightarrow{P'_{23}P'_{13}}$ of each surgical instrument respectively. The direction of the arrow represents the direction of the connecting line $\overrightarrow{P'_{23}P'_{13}}$. Preferably, the length ratio of the three line segments with arrows is equal to the length ratio of the three connecting lines $\overrightarrow{P'_{23}P'_{13}}$. Then, the computing unit allocates the first display frame, and three line segments with arrows are placed in the first display frame as the graphical information of the three connecting lines $\overrightarrow{P'_{23}P'_{13}}$, and the graphical information is sent to the display unit. Preferably, the computing unit further allocates the second display frame in which the image information of the operation environment collected by the endoscope 5 in real time is loaded.

Referring to FIG. 3, a human-machine interface of the display unit 3 has a plurality of graphical display windows, wherein a graphical display window 31 displays information loaded in the second display frame, that is, displays image information of the surgical environment collected by the endoscope 5 in real time. Another graphical display window 32 displays the information loaded in the first display frame, that is, displays the position information of the three surgical instruments processed by the computing unit 4 in a graphical mode. Preferably, an optional graphical display window 33 can display other operation-related information sent by the computing unit 4.

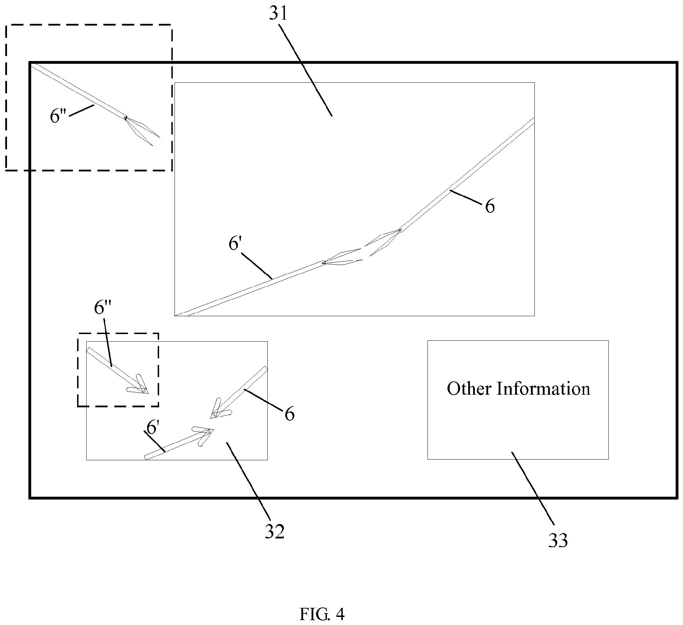

As shown in FIG. 4, when the surgical instrument 6" is not in the field of view of the endoscope, the surgeon can obtain the position information of three surgical instruments, in particular, the position of the surgical instrument 6" relative to other surgical instruments 6, 6' through the graphical display window 32. For ease of understanding, the position of a surgical instrument 6", which is not captured by the endoscope 5, at the upper left corner of FIG. 4 is indicated by a dashed box herein, and represents the position of the surgical instrument 6" relative to the center of the field of view of endoscope in the actual surgical environment. In the graphical display window 32, the three surgical instruments are shown with the first identifier, including the first identifier of the surgical instrument 6" that is not in the field of view of the endoscope 5 within the dashed box at the upper left corner, so that the position relationship between the various surgical instruments is clearly known.

Further, the surgical robot system further includes a control switch communicatively connected to the computing unit 4. When the control switch is turned on, the computing unit 4 performs graphical processing on the position information of the surgical instrument in the endoscope lens plane, and places the obtained graphical information in the first display frame, and then the first display frame is sent to the display unit for display. In this way, the surgeon can decide whether or not to display relevant information.

In some embodiments, the surgical robot system automatically identifies a surgical instrument that is not in the field of view of the endoscope, and specifically displays graphical information of the surgical instrument that is not in the field of view of the endoscope. Specifically, the surgical instruments 6, 6', 6" are provided with identification information, and different surgical instruments are provided with different identification information. For example, the identification information is in a color different from the surgical environment, and is placed at the terminal of the surgical instrument. The computing unit obtains the image information of the surgical environment collected by the endoscope 5 in real time, and then identifies the identification information. If no specific identification information is found, it is indicated that the corresponding surgical instrument is not in the field of view of the endoscope. In this case, the computing unit 4 obtains the position information of the surgical instrument in the endoscope lens plane according to the foregoing method, and when the position information of the surgical instrument is graphically processed, the selected first identifier is different from the first identifiers of other surgical instruments for distinguishing. For example, in the embodiment shown in FIG. 4, the surgical instrument 6" is not within the field of view of the endoscope, and the computing unit 4 uses a dashed line segment with an arrow as the first identifier of the surgical instrument 6". Therefore, in the graphical display window 32, a dashed line segment with an arrow and two solid line segments with arrows are shown.

In some embodiments, the feature point only includes a first feature point. The first feature point may be a surgical instrument endpoint b. For example, the position information of the surgical instrument in the endoscope lens plane is described by a connecting line $\overrightarrow{O_3P'_{13}}$ between the point determined by the projection coordinate value $P'_{13}$ of the first coordinate value $P_{13}$ and the origin $O_3$ of the third coordinate system cys3.

Further, the computing unit 4 obtains the image information of the surgical environment collected by the endoscope 5 in real time, while graphically processing the obtained position information of the surgical instrument in the endoscope lens plane to obtain the graphical information. The computing unit allocates a third display frame, superimposes the graphical information of the surgical instrument with the image information of the surgical environment collected by the endoscope, and then provides the superimposed information in the third display frame, and the third display frame is transmitted to the display unit 3 for display. Specifically, the method for identifying a surgical instrument that is not in the field of view is similar to the foregoing identification method. The position information of the surgical instrument in the endoscope lens plane is described by a connecting line

$$\overline{O_3P'_{13}}$$

between the point determined by the projection coordinate value $P'_{13}$ of the first coordinate value $P_{13}$ and the origin $O_3$ of the third coordinate system cys3. Therefore, the computing unit 4 uses the second identifier

$$\overline{O_3P'_{13}}$$

to indicate the direction of the connecting line

. Preferably, the second identifier is also used to indicate

$$\overline{O_3P'_{13}}$$

the length of the connecting line . Alternatively, the computing unit 4 uses the second identifier to indicate only the direction of the connecting line $\overline{O_3P'_{13}}$ , and uses numbers and/or characters to indicate the length of

the connecting line $\overline{O_3P'_{13}}$ .

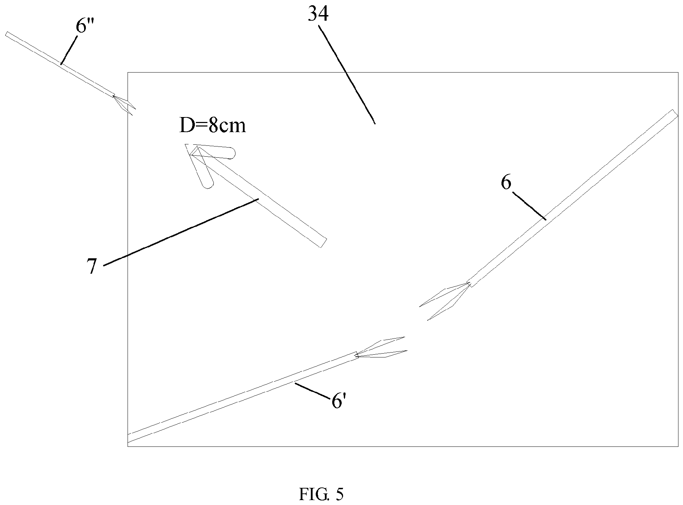

As shown in FIG. 5, the surgical robot system includes three tool arms and corresponding surgical instruments 6, 6', 6". The surgical instrument 6" is identified outside the field of view of the endoscope. The computing unit 4 uses a solid segment with an arrow as the second identifier 7, and "D=8 cm" indicates the length of the con-

necting line $\overline{O_3P'_{13}}$ in accordance with the length of

the connecting line $\overline{O_3P'_{13}}$ of 8 cm. Further, the computing unit 4 superimposes the second identifier 7 with the image information of the surgical environment collected by the endoscope 5 in real time, places the superimposed information into the third display frame, and then the third display frame is transmitted to the display unit 3. A graphical display window 34 of the display unit 3 displays information loaded by the third display frame.

It should be understood by those skilled in the art that in the kinematics equation, the displacement values of known joints can be utilized to calculate the first coordinate value $P_{13}$ and the second coordinate value $P_{23}$. Specifically, the computing unit 4 obtains the displacement values of the respective joints according to the movement state of the joints of the tool arm 2 and the surgical instrument, and obtains the displacement values of the respective joints of the image arm 1 according to the movement state of the joint of the image arm 1, and the obtained displacement values are substituted into the kinematics equation to obtain the first coordinate value $P_{13}$ and the second coordinate value $P_{23}$. In the actual process, the movement of the joint can be either a rotation or a translation.

Generally speaking, a driver 8 and a joint code disc (the joint code disc is, for example, an incremental or absolute encoder) are provided for each joint. The joint code disc is connected to the computing unit 4 and the driver 8. The driver 8 can be used for driving the joints to rotate. The joint code disc can output a pulse signal to the computing unit 4 according to the rotation state of the joint. The computing unit 4 processes the pulse signal to obtain an angle value corresponding to the joint rotation. The computing unit 4 then substitutes the angle value of the joint into the kinematics equation to obtain the first coordinate value $P_{13}$ and the second coordinate value $P_{23}$.

The method for displaying position of a surgical instrument is further described in detail below in conjunction with the surgical robot system.

In some embodiments, the surgical robot system includes three tool arms, one image arm, one display unit, and one computing unit. The terminal of each tool arm is connected to a surgical instrument, and meanwhile, the terminal of the image arm is connected to an endoscope. The computing unit is communicatively connected to the display unit, and the computing unit is communicatively connected to the endoscope on the image arm.

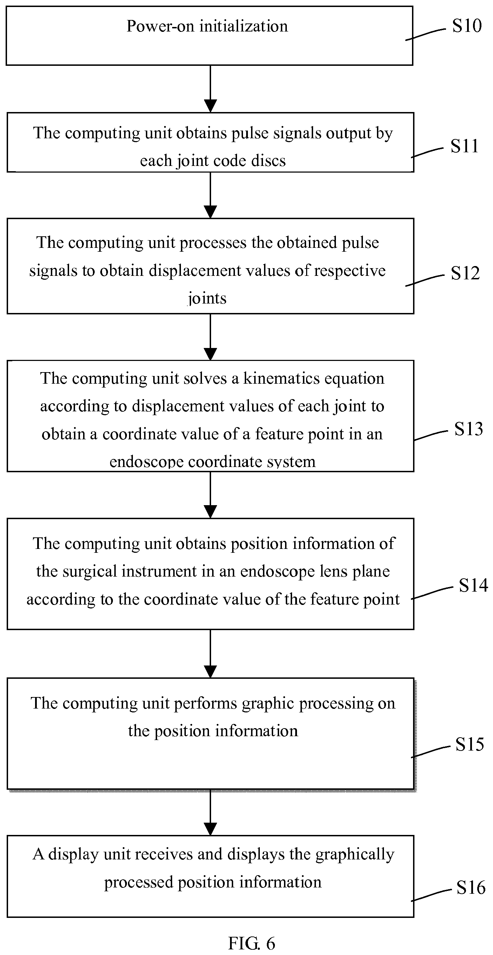

In a method for displaying position, as shown in FIG. 6, after the power-on initialization S 10, the working process of the surgical robot system includes the following steps.

Step S13: a computing unit solves a kinematics equation according to displacement values of respective joints of a tool arm, an image arm and a surgical instrument to obtain a coordinate value of a feature point in an endoscope coordinate system.

Step S14: the computing unit obtains position information of the surgical instrument in an endoscope lens plane according to the coordinate value of the feature point.

Step S15: the computing unit performs graphic processing on the position information.

Step S 16: a display unit receives and displays the graphically processed position information of the surgical instrument in the endoscope lens plane.

Preferably, prior to the step S13, the following steps are further included.

Step S11: the computing unit obtains pulse signals output by respective joint code discs on the tool arm, the image arm and the surgical instrument.

Step S12: the computing unit processes the obtained pulse signals to obtain displacement values of respective joints.

Preferably, step S14 is as follows:

The computing unit obtains a projection of the feature point in the endoscope lens plane according to the coordinate value of the feature point, and further obtains position information of the surgical instrument in the endoscope lens plane. That is, the position information of the surgical instrument in the endoscope lens plane is described by the projection of the feature point in the endoscope lens plane.

More specifically, in an application embodiment such as a urinary surgery, the surgical robot system enters the master-slave mode and is initialized after the surgical robot system is normally turned on by a surgeon. Then, during the operation, it is impossible to know the position of the terminal of the third tool arm when the surgeon cannot see the surgical instrument on the third tool arm in the field of view of the endoscope. Therefore, the surgeon can step on a foot switch, so that the display is switched to the graphical display window in the foregoing embodiments. For example, in one graphical display window of the display, image information of the surgical instruments on two tool arms is displayed, and graphical information shown as the dashed line segment with an arrow appears. The graphical information points toward the surgical instrument on the third tool arm outside the field of view of the endoscope. The direction of the arrow indicates the direction of the surgical instrument on the third tool arm in the field of view of the endoscope, and an annotation at one side of the arrow indicates the horizontal distance from the center of the endoscope coordinate system to the endpoint of the tool arm. In this way, the surgeon knows the position of the third tool arm that is invisible in the field of view of the endoscope and operates the third tool arm smoothly.

The foregoing embodiments describe different display modes of the surgical instrument on the tool arm in detail. Certainly, the present disclosure includes, but is not limited to, the display modes provided in the foregoing embodiments, and any modifications made based on the display modes provided by the foregoing embodiments fall within the protection scope of the present disclosure. Those skilled in the art can draw inferences about other cases from one instance according to the contents of the foregoing embodiments.

In summary, the some embodiments provides a surgical robot system, in particular, in the case where multiple surgical instruments are present, the position information of the surgical instrument can be displayed in an endoscope perspective in a simple and visual manner, so that the position information of the lost surgical instrument can be displayed in a simple and visual manner in the field of view of the endoscope when the surgeon cannot directly obtain one or more surgical instruments in the field of view of the endoscope. In this way, the surgeon can clearly find out the positions of the surgical instruments installed at the terminal of all the tool arms during the operation, thereby improving the operation speed and the operation efficiency, especially reducing the probability of touching the vulnerable organs and improving the safety and success rate of the surgical operation. In addition, since the method of the some embodiments only needs to compute the position information of one or two feature points on each surgical instrument, the computing ability requirement for the computing unit is low, and the cost of the entire surgical robot system is low.

The above description is only for the description of preferred embodiments of the present disclosure

**Claims**

1. A surgical robot system, comprising an image arm (1), a tool arm (2), a display unit (3), and a computing unit (4), wherein

   the image arm (1) is configured to connect to an endoscope (5), the endoscope (5) being configured to collect image information, and an endoscope lens plane of the endoscope (5) is defined with an endoscope coordinate system;
   the tool arm (2) is a remote-center-of-motion point mechanism and configured to connect to a surgical instrument (6, 6', 6"), the surgical instrument (6, 6', 6") comprising:

      a first feature point defined with a first coordinate system, the first feature point corresponding to an endpoint (b) of the surgical instrument, and
      a second feature point (c) defined with a second coordinate system, the second feature point corresponding to the position of the remote-center-of-motion point when the surgical instrument (6, 6', 6") is connected to the tool arm (2);
      wherein, when the endoscope (5) is connected to the image arm (1) and when the surgical instrument (6, 6', 6") is connected to the tool arm (2), the relative position and posture between the coordinate systems are preset and described through a transformation matrix stored in the computing unit (4) for subsequent coordinate transformation; and wherein
      the display unit (3) is communicatively connected to the computing unit (4); and
      the computing unit (4) is configured to:

         obtain displacement values of joints of the image arm (1), the tool arm (2), and the surgical instrument (6, 6', 6"),
         solve, by using the displacement values, a kinematics equation to obtain the coordinate values of both the first feature point and the second feature point in the endoscope coordinate system,
         obtain position information of the surgical instrument (6, 6', 6") in the endoscope lens plane according to the coordinate values of both the first feature point and the second feature point, and

perform graphical processing on the position information;

wherein the display unit (3) is configured to receive and display the graphically processed position information on the basis of the position of both the first feature point and the second feature point in the endoscope lens plane.

2. The surgical robot system according to claim 1, wherein the step of performing graphical processing on the position information by the computing unit (4) comprises: using a first identifier to represent the position information of the surgical instrument (6, 6', 6") in the endoscope lens plane.

3. The surgical robot system according to claim 2, wherein the surgical instrument (6, 6', 6") has identification information; the computing unit (4) obtains image information collected by the endoscope (5), and identifies whether the surgical instrument (6, 6', 6") is in the field of view of the endoscope (5) according to the identification information of the surgical instrument (6, 6', 6").

4. The surgical robot system according to claim 3, comprising at least two surgical instruments (6, 6', 6"), wherein different surgical instruments (6, 6', 6") have different identification information; and the first identifier of one or more surgical instruments (6, 6', 6") which are not in the field of view of the endoscope (5) is different from the first identifier of one or more surgical instruments (6, 6', 6") which are in the field of view of the endoscope (5).

5. The surgical robot system according to claim 2 or 4, wherein the position information of the surgical instrument (6, 6', 6") in the endoscope coordinate system is described by a vector line, wherein a starting point of the vector line is an origin of the endoscope coordinate system, and an end point of the vector line is a projection of the first feature point in the endoscope coordinate system in the endoscope lens plane; and the first identifier represents a direction of the vector line.

6. The surgical robot system according to claim 2 or 4, wherein the position information of the surgical instrument (6, 6', 6") in the endoscope coordinate system is described by a vector line, wherein a starting point of the vector line is a projection of the second feature point in the endoscope coordinate system in the endoscope lens plane, and an end point of the vector line is a projection of the first feature point in the endoscope coordinate system in the endoscope lens plane; and the first identifier represents a direction of the vector line.

7. The surgical robot system according to claim 5 or 6, comprising at least two surgical instruments (6, 6', 6"), wherein a length ratio between the first identifiers of the at least two surgical instruments (6, 6', 6") is equal to a length ratio between the vector lines.

8. The surgical robot system according to claim 2 or 4, wherein the step of performing graphical processing on the position information by the computing unit (4) further comprises:

allocating a first display frame, and loading the first identifier into the first display frame; and sending the first display frame to the display unit (3).

9. The surgical robot system according to claim 1, wherein the step of performing graphical processing on the position information by the computing unit (4) comprises:

using a second identifier (7) to represent the position information; allocating, by the computing unit (4), a third display frame; and placing information obtained by superimposing the second identifier (7) and the image information collected by the endoscope (5) into the third display frame; and sending the third display frame to the display unit (3).

10. The surgical robot system according to claim 9, wherein the position information of the surgical instrument (6, 6', 6") in the endoscope coordinate system is described by a vector line, and the second identifier (7) represents a direction of the vector line,

wherein a starting point of the vector line is an origin of the endoscope coordinate system, and an end point of the vector line is a projection of the first feature point in the endoscope coordinate system in the endoscope lens plane, or the starting point of the vector line is a projection of the second feature point in the endoscope coordinate system in the endoscope lens plane, and the end point of the vector line is a projection of the first feature point in the endoscope coordinate system in the endoscope lens plane.

11. The surgical robot system according to claim 10, wherein the step of performing graphical processing on the position information by the computing unit (4) further comprises: representing the length of the vector line by numbers and/or characters.

12. The surgical robot system according to claim 1, further comprising a control switch, wherein the control switch is communicatively connected to the computing unit (4) and configured to control whether to display the graphically processed position information on the display unit (3).

13. A method of using a surgical robot system according to claim 1 for displaying the position of a surgical instrument (6, 6', 6"), comprising:

solving, by a computing unit (4) according to displacement values of joints of a tool arm (2), an image arm (1) and a surgical instrument (6, 6', 6"), a kinematics equation to obtain in an endoscope coordinate system:

a coordinate value of a first feature point corresponding to an endpoint (b) of the surgical instrument, and
a coordinate value of a second feature point (c) corresponding to the position of a remote-center-of-motion point;

obtaining, by the computing unit (4), position information of the surgical instrument (6, 6', 6") in an endoscope lens plane according to the coordinate value of both the first feature point and the second feature point;
performing, by the computing unit (4), graphical processing on the position information; and
receiving and displaying, by a display unit (3), the graphically processed position information on the basis of the position of both the first feature point and the second feature point in the endoscope lens plane.

**Patentansprüche**

1. Chirurgisches Robotersystem, umfassend einen Bildarm (1), einen Werkzeugarm (2), eine Anzeigeeinheit (3) und eine Recheneinheit (4), wobei

der Bildarm (1) ausgebildet ist, sich mit einem Endoskop (5) zu verbinden, wobei das Endoskop (5) ausgebildet ist, Bildinformationen zu sammeln, und wobei eine Endoskoplinsenebene des Endoskops (5) mit einem Endoskopkoordinatensystem definiert ist;
wobei der Werkzeugarm (2) ein Punktmechanismus mit entferntem Bewegungszentrum ist und ausgebildet ist, sich mit einem chirurgischen Instrument (6, 6', 6") zu verbinden, wobei das chirurgische Instrument (6, 6', 6") umfasst:

einen mit einem ersten Koordinatensystem definierten ersten Merkmalspunkt, wobei der erste Merkmalspunkt einem Endpunkt (b) des chirurgischen Instruments entspricht, und

einen mit einem zweiten Koordinatensystem definierten zweiten Merkmalspunkt (c), wobei der zweite Merkmalspunkt der Position des entfernten Bewegungszentrumspunkts entspricht, wenn das chirurgische Instrument (6, 6', 6") mit dem Werkzeugarm (2) verbunden ist;
wobei, wenn das Endoskop (5) mit dem Bildarm (1) verbunden ist und wenn das chirurgische Instrument (6, 6', 6") mit dem Werkzeugarm (2) verbunden ist, die relative Position und Ausrichtung zwischen den Koordinatensystemen voreingestellt sind und durch eine in der Recheneinheit (4) gespeicherte Transformationsmatrix für eine anschließende Koordinatentransformation beschrieben ist; und wobei
die Anzeigeeinheit (3) mit der Recheneinheit (4) kommunikativ verbunden ist; und
die Recheneinheit (4) ausgebildet ist:

Verschiebungswerte der Gelenke des Bildarms (1), des Werkzeugarms (2) und des chirurgischen Instruments (6, 6', 6") zu erhalten,
unter Verwendung der Verschiebungswerte eine kinematische Gleichung zu lösen, um die Koordinatenwerte sowohl des ersten Merkmalspunkts als auch des zweiten Merkmalspunkts in dem Endoskopkoordinatensystem zu erhalten,
Positionsinformationen des chirurgischen Instruments (6, 6', 6") in der Endoskoplinsenebene gemäß den Koordinatenwerten sowohl des ersten Merkmalspunkts als auch des zweiten Merkmalspunkts zu erhalten, und
eine grafische Verarbeitung der Positionsinformationen durchzuführen;
wobei die Anzeigeeinheit (3) ausgebildet ist, die grafisch verarbeiteten Positionsinformationen basierend auf der Position sowohl des ersten Merkmalspunkts als auch des zweiten Merkmalspunkts in der Endoskoplinsenebene zu empfangen und anzuzeigen.

2. Chirurgisches Robotersystem nach Anspruch 1, wobei der Schritt des Durchführens der grafischen Verarbeitung der Positionsinformationen durch die Recheneinheit (4) umfasst: Verwenden eines ersten Identifikators zur Repräsentation der Positionsinformationen des chirurgischen Instruments (6, 6', 6") in der Endoskoplinsenebene.

3. Chirurgisches Robotersystem nach Anspruch 2, wobei das chirurgische Instrument (6, 6', 6") Identifikationsinformationen aufweist; wobei die Recheneinheit (4) durch das Endoskop (5) gesammelte Bildinformationen erhält und gemäß der Identifikationsinformationen des chirurgischen Instruments (6, 6', 6") identifiziert, ob sich das chirurgische Instrument (6, 6', 6") in dem Sichtfeld des Endoskops (5) befindet.

4. Chirurgisches Robotersystem nach Anspruch 3, umfassend mindestens zwei chirurgische Instrumente (6, 6', 6"), wobei unterschiedliche chirurgische Instrumente (6, 6', 6") unterschiedliche Identifikationsinformationen aufweisen; und wobei der erste Identifikator eines oder mehrerer chirurgischer Instrumente (6, 6', 6"), die sich nicht im Sichtfeld des Endoskops (5) befinden, sich von dem ersten Identifikator eines oder mehrerer chirurgischer Instrumente (6, 6', 6") unterscheidet, die sich im Sichtfeld des Endoskops (5) befinden.

5. Chirurgisches Robotersystem nach Anspruch 2 oder 4, wobei die Positionsinformation des chirurgischen Instruments (6, 6', 6") in dem Endoskopkoordinatensystem durch eine Vektorlinie beschrieben wird, wobei ein Startpunkt der Vektorlinie ein Ursprung des Endoskopkoordinatensystems und ein Endpunkt der Vektorlinie eine Projektion des ersten Merkmalspunkts in dem Endoskopkoordinatensystem in die Endoskoplinsenebene ist; und wobei der erste Identifikator eine Richtung der Vektorlinie repräsentiert.

6. Chirurgisches Robotersystem nach Anspruch 2 oder 4, wobei die Positionsinformation des chirurgischen Instruments (6, 6', 6") in dem Endoskopkoordinatensystem durch eine Vektorlinie beschrieben wird, wobei ein Startpunkt der Vektorlinie eine Projektion des zweiten Merkmalspunkts in dem Endoskopkoordinatensystem in die Endoskoplinsenebene und ein Endpunkt der Vektorlinie eine Projektion des ersten Merkmalspunkts in dem Endoskopkoordinatensystem in die Endoskoplinsenebene ist; und wobei der erste Identifikator eine Richtung der Vektorlinie repräsentiert.

7. Chirurgisches Robotersystem nach Anspruch 5 oder 6, umfassend mindestens zwei chirurgische Instrumente (6, 6', 6"), wobei ein Längenverhältnis zwischen dem ersten Identifikator der mindestens zwei chirurgischen Instrumente (6, 6', 6") gleich einem Längenverhältnis zwischen den Vektorlinien ist.

8. Chirurgisches Robotersystem nach Anspruch 2 oder 4, wobei der Schritt des Durchführens der grafischen Verarbeitung der Positionsinformationen durch die Recheneinheit (4) ferner umfasst:

    Zuweisen eines ersten Anzeigerahmens und Laden des ersten Identifikators in den ersten Anzeigerahmen; und

    Senden des ersten Anzeigerahmens an die Anzeigeeinheit (3).

9. Chirurgisches Robotersystem nach Anspruch 1, wobei der Schritt des Durchführens der grafischen Verarbeitung der Positionsinformationen durch die Recheneinheit (4) umfasst:

    Verwenden eines zweiten Identifikators (7) zur Repräsentation der Positionsinformation; Zuweisen eines dritten Anzeigerahmens durch die Recheneinheit (4); und Platzieren einer durch Überlagerung des zweiten Identifikators (7) und der von dem Endoskop (5) gesammelten Bildinformation erhaltenen Information in dem dritten Anzeigerahmen; und
    Senden des dritten Anzeigerahmens an die Anzeigeeinheit (3).

10. Chirurgisches Robotersystem nach Anspruch 9, wobei die Positionsinformation des chirurgischen Instruments (6, 6', 6") in dem Endoskopkoordinatensystem durch eine Vektorlinie beschrieben wird, und wobei der zweite Identifikator (7) eine Richtung der Vektorlinie repräsentiert,

    wobei ein Startpunkt der Vektorlinie ein Ursprung des Endoskopkoordinatensystems ist, und wobei ein Endpunkt der Vektorlinie eine Projektion des ersten Merkmalspunkts in dem Endoskopkoordinatensystem in die Endoskoplinsenebene ist, oder
    wobei der Startpunkt der Vektorlinie eine Projektion des zweiten Merkmalspunkts in dem Endoskopkoordinatensystem in die Endoskoplinsenebene ist, und wobei der Endpunkt der Vektorlinie eine Projektion des ersten Merkmalspunkts in dem Endoskopkoordinatensystem in die Endoskoplinsenebene ist.

11. Chirurgisches Robotersystem nach Anspruch 10, wobei der Schritt des Durchführens der grafischen Verarbeitung der Positionsinformationen durch die Recheneinheit (4) ferner umfasst: Repräsentieren der Länge der Vektorlinie durch Zahlen und/oder Zeichen.

12. Chirurgisches Robotersystem nach Anspruch 1, ferner umfassend einen Steuerschalter, wobei der Steuerschalter mit der Recheneinheit (4) kommunikativ verbunden ist und ausgebildet ist, zu steuern, ob die grafisch verarbeiteten Positionsinformationen auf der Anzeigeeinheit (3) angezeigt werden sollen.

13. Verfahren zur Verwendung eines chirurgischen Robotersystems nach Anspruch 1 zur Anzeige der Position eines chirurgischen Instruments (6, 6', 6"), umfassend:

Lösen einer kinematischen Gleichung entsprechend Verschiebungswerten der Gelenke eines Werkzeugarms (2), eines Bildarms (1) und eines chirurgischen Instruments (6, 6', 6") durch eine Recheneinheit (4), um in einem Endoskopkoordinatensystem zu erhalten:

einen Koordinatenwert eines ersten Merkmalspunkts, der einem Endpunkt (b) des chirurgischen Instruments entspricht, und einen Koordinatenwert eines zweiten Merkmalspunkts (c), der der Position eines entfernten Bewegungszentrumspunkts entspricht;

Erhalten von Positionsinformationen des chirurgischen Instruments (6, 6', 6") in einer Endoskoplinsenebene gemäß den Koordinatenwerten sowohl des ersten Merkmalspunkts als auch des zweiten Merkmalspunkts durch die Recheneinheit (4);

Durchführen einer grafischen Verarbeitung der Positionsinformationen durch die Recheneinheit (4); und

Empfangen und Anzeigen der grafisch verarbeiteten Positionsinformationen basierend auf der Position sowohl des ersten Merkmalspunkts als auch des zweiten Merkmalspunkts in der Endoskoplinsenebene durch eine Anzeigeeinheit (3).

**Revendications**

1. Système de robot chirurgical, comprenant un bras d'image (1), un bras d'outil (2), une unité d'affichage (3) et une unité de calcul (4), dans lequel

le bras d'image (1) est configuré pour se connecter à un endoscope (5), l'endoscope (5) étant configuré pour collecter des informations d'image, et un plan de lentille d'endoscope de l'endoscope (5) est défini avec un système de coordonnées d'endoscope ;

le bras d'outil (2) est un mécanisme de point de mouvement à centre éloigné et configuré pour se connecter à un instrument chirurgical (6, 6', 6"), l'instrument chirurgical (6, 6', 6") comprenant :

un premier point caractéristique défini avec un premier système de coordonnées, le premier point caractéristique correspondant à un point final (b) de l'instrument chirurgical, et

un deuxième point caractéristique (c) défini avec un deuxième système de coordonnées, le deuxième point caractéristique correspondant à la position du point de centre de mouvement éloigné lorsque l'instrument chirurgical (6, 6', 6") est connecté au bras d'outil (2) ;

dans lequel, lorsque l'endoscope (5) est connecté au bras d'image (1) et lorsque l'instrument chirurgical (6, 6', 6") est connecté au bras d'outil (2), la position et la posture relatives entre les coordonnées les systèmes sont prédéfinis et décrits par l'intermédiaire d'une matrice de transformation stockée dans l'unité informatique (4) pour une transformation de coordonnées ultérieure ; et dans lequel

l'unité d'affichage (3) est connectée en communication à l'unité informatique (4) ; et l'unité de calcul (4) est configurée pour :

obtenir des valeurs de déplacement des articulations du bras d'image (1), du bras d'outil (2) et de l'instrument chirurgical (6, 6', 6"),

résoudre, à l'aide des valeurs de déplacement, une équation cinématique pour obtenir les valeurs de coordonnées du premier point caractéristique et du deuxième point caractéristique dans le système de coordonnées de l'endoscope,

obtenir des informations de position de l'instrument chirurgical (6, 6', 6") dans le plan de la lentille de l'endoscope en fonction des valeurs de coordonnées à la fois du premier point caractéristique et du deuxième point caractéristique, et effectuer un traitement graphique sur les informations de position ;

étant configurée pour recevoir et afficher les informations de position traitées graphiquement sur la base de la position à la fois du premier point caractéristique et du deuxième point caractéristique dans le plan de la lentille de l'endoscope.

2. Système de robot chirurgical selon la revendication 1, dans lequel l'étape d'exécution d'un traitement graphique sur les informations de position par l'unité informatique (4) comprend : utiliser un premier identifiant pour représenter les informations de position de l'instrument chirurgical (6, 6', 6") dans le plan de la lentille de l'endoscope.

3. Système de robot chirurgical selon la revendication 2, dans lequel l'instrument chirurgical (6, 6', 6") possède des informations d'identification ; l'unité informatique (4) obtient des informations d'image collectées par l'endoscope (5) et identifie si l'instrument chirurgical (6, 6', 6") se trouve dans le champ de vision de l'endoscope (5) en fonction des informations d'identification de l'instrument chirurgical (6, 6', 6").

4. Système de robot chirurgical selon la revendication 3, comprenant au moins deux instruments chirurgicaux (6, 6', 6"), dans lequel différents instruments chirurgicaux (6, 6', 6") ont des informations d'identification différentes ; et le premier identifiant d'un ou plusieurs instruments chirurgicaux (6, 6', 6") qui ne se trouvent pas dans le champ de vision de l'endoscope (5) est différent du premier identifiant d'un ou plusieurs instruments chirurgicaux (6, 6', 6") qui se trouvent dans le champ de vision de l'endoscope (5).

5. Système de robot chirurgical selon la revendication 2 ou 4, dans lequel les informations de position de l'instrument chirurgical (6, 6', 6") dans le système de coordonnées de l'endoscope sont décrites par une ligne vectorielle, dans lequel un point de départ du vecteur la ligne est une origine du système de coordonnées de l'endoscope, et un point final de la ligne vectorielle est une projection du premier point caractéristique dans le système de coordonnées de l'endoscope dans le plan de la lentille de l'endoscope ; et le premier identifiant représente une direction de la ligne vectorielle.

6. Système de robot chirurgical selon la revendication 2 ou 4, dans lequel les informations de position de l'instrument chirurgical (6, 6', 6") dans le système de coordonnées de l'endoscope sont décrites par une ligne vectorielle, dans lequel un point de départ du vecteur la ligne est une projection du deuxième point caractéristique dans le système de coordonnées de l'endoscope dans le plan de la lentille de l'endoscope, et un point final de la ligne vectorielle est une projection du premier point caractéristique dans le système de coordonnées de l'endoscope dans le plan de la lentille de l'endoscope ; et le premier identifiant représente une direction de la ligne vectorielle.

7. Système de robot chirurgical selon la revendication 5 ou 6, comprenant au moins deux instruments chirurgicaux (6, 6', 6"), dans lequel un rapport de longueur entre les premiers identifiants des au moins deux instruments chirurgicaux (6, 6', 6") est égal à un rapport de longueur entre les lignes vectorielles.

8. Système robot chirurgical selon la revendication 2 ou 4, dans lequel l'étape d'exécution d'un traitement graphique sur les informations de position par l'unité de calcul (4) comprend en outre :

attribuer un premier cadre d'affichage et charger le premier identifiant dans le premier cadre d'affichage ; et
envoyer la première trame d'affichage à l'unité d'affichage (3).

9. Système de robot chirurgical selon la revendication 1, dans lequel l'étape d'exécution d'un traitement graphique sur les informations de position par l'unité de calcul (4) comprend :

utiliser un deuxième identifiant (7) pour représenter les informations de position ; attribuer, par l'unité informatique (4), une troisième trame d'affichage ; et placer les informations obtenues en superposant le deuxième identifiant (7) et les informations d'image collectées par l'endoscope (5) dans le troisième cadre d'affichage ; et envoyer la troisième trame d'affichage à l'unité d'affichage (3).

10. Système de robot chirurgical selon la revendication 9, dans lequel les informations de position de l'instrument chirurgical (6, 6', 6") dans le système de coordonnées de l'endoscope sont décrites par une ligne vectorielle, et le deuxième identifiant (7) représente un direction de la ligne vectorielle,

dans lequel un point de départ de la ligne vectorielle est une origine du système de coordonnées de l'endoscope, et un point final de la ligne vectorielle est une projection du premier point caractéristique dans le système de coordonnées de l'endoscope dans le plan de la lentille de l'endoscope, ou
le point de départ de la ligne vectorielle est une projection du deuxième point caractéristique dans le système de coordonnées de l'endoscope dans le plan de la lentille de l'endoscope, et le point final de la ligne vectorielle est une projection du premier point caractéristique dans le système de coordonnées de l'endoscope dans l'endoscope plan de la lentille.

11. Système de robot chirurgical selon la revendication 10, dans lequel l'étape d'exécution d'un traitement graphique sur les informations de position par l'unité informatique (4) comprend en outre : représenter la longueur de la ligne vectorielle par des nombres et/ou des caractères.

12. Système de robot chirurgical selon la revendication 1, comprenant en outre un commutateur de commande, dans lequel le commutateur de commande est connecté de manière communicative à l'unité informatique (4) et configuré pour contrôler s'il faut afficher les informations de position traitées graphiquement sur l'unité d'affichage (3).

13. Procédé d'utilisation d'un système de robot chirurgical selon l'une quelconque des revendications précédentes pour afficher la position d'un instrument chirurgical (6, 6', 6"), comprenant :

résoudre, par une unité de calcul (4) en fonction des valeurs de déplacement des articulations d'un bras d'outil (2), d'un bras d'image (1) et d'un instrument chirurgical (6, 6', 6"), une équation cinématique pour obtenir en un système de coordonnées d'endoscope :

une valeur de coordonnée d'un premier point caractéristique correspondant à un point final (b) de l'instrument chirurgical, et

une valeur de coordonnée d'un deuxième point caractéristique (c) correspondant à la position d'un point de centre de mouvement éloigné ;

obtenir, au moyen de l'unité de calcul (4), des informations de position de l'instrument chirurgical (6, 6', 6") dans un plan de lentille d'endoscope en fonction de la valeur de coordonnée à la fois du premier point caractéristique et du deuxième point caractéristique ;

effectuer, par l'unité de calcul (4), un traitement graphique sur les informations de position ; et

recevoir et afficher, par une unité d'affichage (3), les informations de position traitées graphiquement sur la base de la position à la fois du premier point caractéristique et du deuxième point caractéristique dans le plan de la lentille de l'endoscope.

FIG. 1

FIG. 2

FIG. 3

FIG. 4

FIG. 5

```
┌─────────────────────────────────────────┐
│         Power-on initialization          │ ── S10
└─────────────────────────────────────────┘
                    │
                    ▼
┌─────────────────────────────────────────┐
│  The computing unit obtains pulse        │
│  signals output by each joint code discs │ ── S11
└─────────────────────────────────────────┘
                    │
                    ▼
┌─────────────────────────────────────────┐
│  The computing unit processes the        │
│  obtained pulse signals to obtain        │ ── S12
│  displacement values of respective joints│
└─────────────────────────────────────────┘
                    │
                    ▼
┌─────────────────────────────────────────┐
│  The computing unit solves a kinematics  │
│  equation according to displacement      │
│  values of each joint to obtain a        │ ── S13
│  coordinate value of a feature point in  │
│  an endoscope coordinate system          │
└─────────────────────────────────────────┘
                    │
                    ▼
┌─────────────────────────────────────────┐
│  The computing unit obtains position     │
│  information of the surgical instrument  │
│  in an endoscope lens plane according to │ ── S14
│  the coordinate value of the feature     │
│  point                                   │
└─────────────────────────────────────────┘
                    │
                    ▼
┌─────────────────────────────────────────┐
│  The computing unit performs graphic     │
│  processing on the position information  │ ── S15
└─────────────────────────────────────────┘
                    │
                    ▼
┌─────────────────────────────────────────┐
│  A display unit receives and displays    │
│  the graphically processed position      │ ── S16
│  information                             │
└─────────────────────────────────────────┘
```

FIG. 6

**EP 3 636 194 B1**

**REFERENCES CITED IN THE DESCRIPTION**

**Patent documents cited in the description**

- CN 102448680 A
- EP 2046538 A2